# EUROPÄISCHE PATENTSCHRIFT

(11) **EP 3 292 769 B1**
(45) Veröffentlichungstag und Bekanntmachung des Hinweises auf die Patenterteilung: **17.04.2019**
(21) Anmeldenummer: 17189932.1
(22) Anmeldetag: 07.09.2017
(51) Int. Cl.: A23J 3/04, A23J 3/20, A23J 3/14, A23J 3/22, A23L 31/00

(54) **FLEISCHERSATZ- ODER FLEISCHIMITATPRODUKT**
MEAT SUBSTITUTE OR MEAT IMITATION PRODUCT
PRODUIT DE REMPLACEMENT OU D'IMITATION DE VIANDE

(30) Priorität: 09.09.2016 AT 508012016
(43) Veröffentlichungstag der Anmeldung: 14.03.2018
(73) Patentinhaber: Neuburger Fleischlos GmbH, 4161 Ulrichsberg (AT)
(72) Erfinder: Neuburger, Hermann, 4161 Ulrichsberg (AT); Neuburger, Thomas, 4161 Ulrichsberg (AT)
(74) Vertreter: Hadeyer, Christian

(56) Entgegenhaltungen:
- DE-T2- 69 604 562
- KR-A- 20140 135 041
- NL-C2- 1 031 632
- US-A1- 2009 148 558
- DATABASE GNPD [Online] MINTEL; 1. Januar 2011 (2011-01-01), "Seaweed Veggie Balls", XP002777261, gefunden im http://www.gnpd.com/sinatra/recordpage/164 4238/from_search/blfOjdzhDE/?page=1 Database accession no. 1644238

## Beschreibung

Die Erfindung betrifft ein Verfahren zur Herstellung von Fleischersatz- oder Fleischimitatprodukten basierend auf Speisepilzen und deren Fruchtkörpern. Die Erfindung betrifft weiters die Produkte, welche durch das Verfahren hergestellt werden und deren Verwendung.

Als Fleischersatz- oder Fleischimitatprodukte werden Produkte verstanden, welche von der Konsistenz bzw. Bissfestigkeit und dem Geschmack von auf Fleisch basierten Produkten möglichst ähnlich sind, allerdings keine Fleischrohstoffe beinhalten. Dadurch können Vegetarier und Veganer auch ohne den Konsum von Fleisch auf Produkte zurückgreifen, welche in der Regel ausschließlich mit Fleisch hergestellt werden.

Gängige Fleischersatz- oder Fleischimitatprodukte basieren auf pflanzlichen Rohstoffen (z.B. Seitan aus Weizengluten oder Tofu aus Sojamilch), tierischen Rohstoffen (z.B. auf Milchbasis) oder mikrobiologischen Rohstoffen (z.B. Myzel von Schimmelpilzen) und können teilweise nur unter hohem industriellen Aufwand produziert werden.

Seitan wird aus Weizenproteinen (Gluten) hergestellt und durch die Behandlung mit verschiedenen Gewürzen industriell zu vegetarischen/veganen Produkten verarbeitet (z.B. Seitan-Würste oder Seitan-Schnitzel). Die Konsistenz des fertigen Produktes ist sehr zäh, was ihn von der typischen Fleischkonsistenz unterscheidet.

Tofu wird durch Gerinnung des Eiweißes aus Sojamilch und anschließender Pressung hergestellt. Das fertige Produkt ist in der Konsistenz als zart zu beschreiben, weshalb der Verzehr an weichen Käse erinnert. Der Geschmack von Tofu ist neutral, weswegen dieses Produkt meist in Kombination mit Sojasauce oder anderen Gewürzmitteln verzehrt wird.

Für Sojagranulat werden Sojabohnen geschrotet und entweder direkt zum Verzehr als Hackfleisch-Ersatz verwendet oder weiter industriell zu Fleischimitatprodukten verarbeitet.

In der WO2007139321 (A1) wird ein Fleischimitat auf Basis von Schimmelpilzmyzelien beschrieben, welche kultiviert und zu fleischähnlichen Produkten weiterverarbeitet werden können. Dabei werden ausschließlich die Pilzmyzelien herangezogen. Nachteilig ist die Verwendung eines mit Pilzmyzelien bzw. eines von Pilzmyzelien durchsetzten Substrats als Rohstoff, woraus die Probleme resultieren, dass es sich bei derart hergestellten Produkten wahrscheinlich um sogenannte "Novel-food" Produkte handelt und dass eine gleichbleibende Qualität der Pilzmyzelien bzw. des mit Pilzmyzelien durchsetzen Substrats schwer erreichbar bzw. kontrollierbar ist.

In der WO03061400 (A1) wird ein Fleischimitat auf Milchbasis beschrieben, welches durch Fällung von Milchprodukten mit Metallkationen und anschließender homogener Vermischung mit Wasser, faserigen Rohstoffen und Gewürzen zu einem Fertigfleischersatzprodukt hergestellt wird.

Die NL1031632 (C2) beschreibt ein Verfahren zur Herstellung eines Lebensmittelprodukts auf Basis von Protein/Stärke, welche Teile von Fleischerzeugnissen und Pilzen in einem Verhältnis von 1:1 bis 1:9 enthält. Da das Lebensmittelprodukt Fleischerzeugnisse (z.B. Rindfleisch, Schweinefleisch, Lammfleisch, etc.) enthält, stellt dieses jedoch kein Fleischersatz- oder Fleischimitatprodukt im eigentlichen Sinn, jedenfalls kein fleischloses Fleischersatz- oder Fleischimitatprodukt, dar.

Die EP1254604 (A1) beschreibt ein Halbfertigprodukt zur Herstellung eines Fleischersatz- oder Fleischimitatprodukts, welches aus Pilzen (z.B. Pleurotus spp.), Faserstoffen, Proteinen, Bindemittel, Wasser und ggf. Öl besteht. Als Protein wird Erbsen-Protein eingesetzt. Da die Zutaten und insbesondere auch die Pilze mit einem Fleisch-Kutter ("cuttering device") zerkleinert werden, liegen diese als feine Paste vor.

Die KR20140135041 (A) offenbart ein Analog-Fleisch auf Basis von Reis und Pilzen (z.B. Shiitake), wobei die Pilze pulverisiert werden und Reis als Mehl eingesetzt wird, sodass sich eine sehr feine Textur ergibt.

Bei der DE69604562 (A1) wird in einem ersten Schritt eine Paste aus essbaren faserartigen Pilzen und gelierendem essbaren Hydrokolloid hergestellt. Durch Gelieren wird aus der Paste eine feste Masse, welche dann gefroren und zerstückelt wird. Der stückige Anteil ist somit nicht durch den Pilz gebildet, sondern durch eine gelierte feste Paste, welche pastösen Pilz enthält.

Die Aufgabe der gegenständlichen Erfindung ist es, Fleischersatz- oder Fleischimitatprodukte bereitzustellen, welche aus erprobten und in großen Mengen verfügbaren Rohstoffen herstellbar sind, wobei die fertigen Produkte eine Textur vergleichbar zu Fleisch und Fleischerzeugnissen aufweisen sollen. Dabei soll unter Anwendung eines Herstellungsverfahrens unter Verwendung desselben Rohstoffs eine breite Palette an Fleischsorten bzw. Fleischprodukten nachbildbar sein. Zur Lösung der Aufgabe wird ein Verfahren nach Anspruch 1 und ein Fleischersatz- oder Fleischimitatprodukt gemäß Anspruch 7 vorgeschlagen.

Beim Verfahren wird ein Fleischersatz- oder Fleischimitatproduktunter Verwendung der Fruchtkörper von Speisepilzen als Hauptrohstoff hergestellt, wobei
- im ersten Schritt die Fruchtkörper zu Stücken zerkleinert werden, wobei zumindest eine Teilmenge der Fruchtkörper auf Stücke mit einer minimalen Erstreckung von 3mm zerkleinert wird,
- im zweiten Schritt die zerkleinerten Fruchtkörper mit weiteren Zutaten umfassend zumindest pflanzliches und/oder tierisches Protein zu einer homogenen Produktionsmasse vermischt werden,
- im dritten Schritt die Produktionsmasse in zumindest eine Form abgefüllt wird und
- im vierten Schritt die Produktionsmasse in der zumindest einen Form erhitzt wird, sodass sich das pflanzliche oder tierische Protein verfestigt,
wobei das Produkt zumindest folgende Zutaten im angegebenen Wertebereich aufweist:
- Fruchtkörper von Speisepilzen im Bereich von 40 bis 90 Gew%
- Pflanzenöl im Bereich von 2 bis 20 Gew%
- eine Stärkequelle, insbesondere gekochter Reis, im Bereich von 2 bis 15 Gew%
- tierische und/oder pflanzliche Proteine im Bereich von 4 bis 15 Gew%
- Salz im Bereich von 1 bis 3 Gew%.

Vorteilhaft ist die Verwendung von Fruchtkörpern von Speisepilzen als Rohstoff, da diese ein gängiges und erprobtes Lebensmittel darstellen und das erfindungsgemäße Produkt somit keiner besonderen Erprobung bzw. Zulassung bedarf.

Der Speisepilz ist im erfindungsgemäß hergestellten Fleischersatz- oder Fleischimitatprodukt mit einem Anteil von 40 bis 90 Gew% enthalten. Bevorzugt werden als Hauptrohstoff die Fruchtkörper von Speisepilzen der Gattung Pleurotus verwendet. Besonders vorteilhaft ist die Verwendung von Fruchtkörpern von Pleurotus eryngii (Brauner Kräuterseitling) als Rohstoff, da diese Pilze gut in großen Mengen züchtbar sind und das Fleisch des Fruchtkörpers im Vergleich zu anderen Pilzen eine eher feste Konsistenz aufweist.

Bevorzugt umfassen die weiteren Zutaten Reis, getrocknetes Hühnereieiweiß, Pflanzenöl, Salz und Gewürze. Vorteilhaft ist, dass alle Zutaten des erfindungsgemäßen Produkts in Bio-Qualität verfügbar sind, sodass das erfindungsgemäße Produkt bevorzugt als Bioprodukt vorliegen kann. Die nach dem gegenständlichen Verfahren hergestellten Fleischersatz- oder Fleischimitatprodukte zeichnen sich durch eine Druckfestigkeit und Textur aus, die vergleichbar mit Fleisch oder Fleischprodukten ist, wobei sich durch das erfindungsgemäße Verfahren aus ein und demselben Rohstoff unterschiedliche Texturen herstellen lassen, um die Texturen unterschiedlicher Fleischerzeugnisse, wie beispielsweise Würste, Schnitzel, Fischstäbchen, Hühnernuggets, Gyros oder Bratstreifen nachbilden zu können. Die Dichte des fertigen Produktes liegt bevorzugt im Bereich zwischen 0,9 bis 1,8 g/cm³.

Das besonders bevorzugte Verfahren umfasst die Schritte:
- (falls nötig) Vorbereitung der Pilze (z.B. Reinigen),
- Zerkleinern der Pilze auf Stücke von 3 bis 15 mm,
- Einfüllen der Pilze in einen Mischer,
- Zugabe der weiteren Zutaten umfassend zumindest pflanzliches und/oder tierisches Eiweiß in den Mischer und Mischen bis eine homogene Produktionsmasse hergestellt ist,
- Portionierung der Produktionsmasse in Formen und
- Erhitzung der Rohmasse in den Formen.

Die Erfindung wird an Hand von Zeichnungen veranschaulicht:
- Fig. 1:: zeigt ein Prozessfließbild des erfindungsgemäßen Verfahrens.
- Fig. 2:: zeigt das erfindungsgemäße Verfahren mit schematischer Darstellung der wesentlichen Anlagenkomponenten.

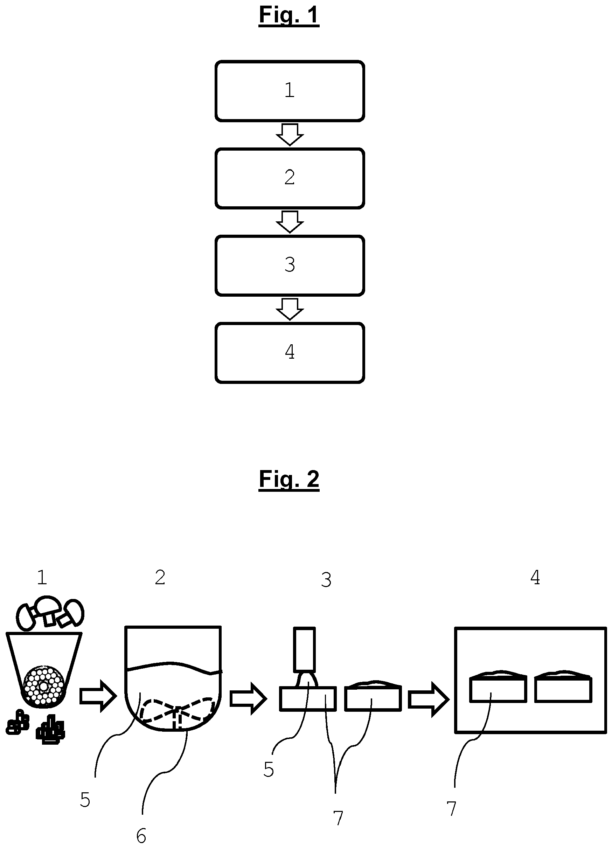

In Fig. 1 werden die einzelnen Verfahrensschritte gezeigt, wobei im ersten Schritt 1 die Zerkleinerung der Fruchtkörper des Pilzes erfolgt, im zweiten Schritt 2 das Mischen der Fruchtkörper mit den weiteren Zutaten erfolgt, im dritten Schritt 3 das Abfüllen der Produktionsmasse in zumindest eine Form 7 erfolgt und im vierten Schritt 4 das Erhitzen der Produktionsmasse in der zumindest einen Form 7.

Im ersten Schritt 1 werden die Fruchtkörper des Speisepilzes zerkleinert. Um eine besonders feste Konsistenz oder Dichte zu erreichen, hat es sich als vorteilhaft herausgestellt, die Fruchtkörper auf Stücke mit einer mittleren Größe von 3 mm bis 15 mm zu zerkleinern. Der Zerkleinerungsschritt erfolgt bevorzugt durch Pressen der Fruchtkörper durch eine Lochscheibe, beispielsweise mit einer Fleischereimaschine (auch Fleischwolf genannt). Dabei werden die gegebenenfalls zuvor grob zerkleinerten Fruchtkörper durch eine Förderschnecke in Richtung der Lochscheibe transportiert, vor welcher sich eine rotierende Messerscheibe befindet. Besonders gute Resultate wurden mit Lochscheiben erzielt, deren Rundlöcher einen Durchmesser von 6 bis 10 mm aufweisen, wobei besonders bevorzugt ein Lochdurchmesser von 8 mm verwendet wird, woraus unregelmäßig geformte und zylindrische Fruchtkörperstücke mit einer max. Erstreckung von ca. 3 bis 15 mm resultieren.

In einer weiteren bevorzugten Ausführungsvariante werden die Fruchtkörper durch Lochscheiben gepresst, die rechteckige, insbesondere quadratische Öffnungen aufweisen, sodass quaderförmige Stücke oder quadratische Säulen resultieren. Die Öffnungen weisen dabei bevorzugt Kantenlängen von 3 bis 9 mm auf, wobei besonders bevorzugt quadratische Öffnungen mit Kantenlänge 6 mm x 6 mm verwendet werden. Die bevorzugte Länge der quadratischen Säulen bzw. quaderförmigen Stücke beträgt 5 bis 25 mm.

Die Fruchtkörper von Speisepilzen weisen einen Bereich von 40 bis 90 Gew% in den Ausführungsvarianten auf. Das Pflanzenöl weist bevorzugt einen Bereich von 2 bis 15 Gew% in den Ausführungsvarianten auf. Der gekochte Reis weist bevorzugt einen Bereich von 2 bis 15 Gew% in den Ausführungsvarianten auf. Das Hühnereieiweiß weist bevorzugt einen Bereich von 4 bis 12 Gew% in den Ausführungsvarianten auf. Salz weist einen Bereich von bevorzugt 1 bis 3 Gew% in den Ausführungsvarianten auf.

In einer besonders bevorzugten Ausführungsvariante werden unterschiedlich zerkleinerte Teilmengen von Fruchtkörpern eingesetzt, sodass das fertige Produkt sowohl Fruchtkörperstücke enthält die durch die Rundlöcher geformt wurden, als auch besagte quaderförmige Stücke. Dabei sind die quaderförmigen Stücke bevorzugt größer ausgeführt, als die durch Rundlöcher geformten Stücke, sodass sich die quaderförmigen Stücke im fertigen Produkt von der sie umgebenden Masse unterscheiden. Dadurch können beispielsweise grobe zerkleinerte Fleischstücke, welche im Brät einer Bratwurst vorliegen, vorteilhaft nachgeahmt werden. Wie in Fig. 2 veranschaulicht, wird im zweiten Schritt 2 die Produktionsmasse 5, bestehend aus zerkleinerten Fruchtkörpern und den weiteren Zutaten, mit einem Mischer 6 für bevorzugt 1 bis 10 Minuten bei einer Temperatur von bevorzugt 10 bis 20 °C homogenisiert, wobei an weiteren Zutaten zumindest eine Stärkequelle (z.B. Reis, insbesondere in Form von gekochtem Reis), eine Ölquelle (z.B. Rapsöl oder Sonnenblumenöl), eine Proteinquelle (z.B. getrocknetes Hühnereieiweiß), Salz und Gewürze zugegeben werden.

Im dritten Schritt 3 wird die Produktionsmasse 5 in zumindest einer Form 7 abgefüllt. Eine bevorzugte Form 7 besteht aus Silikon und weist zum Beispiel 25 bis 30 füllbare Teil-Volumen von je 20 bis 100 cm³ auf. Eine weitere bevorzugte Form 7 besteht aus Kunststoff und liegt in Form einer zu füllenden Wursthülle vor, welche bevorzugt wie bei der Wurstproduktion üblich als Schlauch vorliegt.

Im vierten Schritt 4 wird die Produktionsmasse 5 in der zumindest einen Form 7 für 15 bis 45 Minuten bei einer Temperatur von 90 bis 100 °C erhitzt. Dabei wird durch die Denaturierung der verwendeten Proteinquelle eine Verfestigung der Produktionsmasse erzielt.

### Beispiel 1

In Beispiel 1 wurde das erfindungsgemäße Verfahren dazu verwendet, um ein Fleischersatz- oder Fleischimitatprodukt als Imitat von Schnitzelfleisch herzustellen. Die Zerkleinerung von Kräuterseitlingen erfolgte mit einer Wolfscheibe mit Rundlöchern von 8 mm Durchmesser. Die Produktionsmasse wurde im zweiten Schritt 2 durch Mischen der zerkleinerten Kräuterseitlinge (77 Gew%) mit gekochtem Reis, getrocknetem Hühnereieiweiß, Rapsöl, Salz und Gewürzen erhalten. Die Produktionsmasse wurde im dritten Schritt 3 in eine Silikonform abgefüllt. Im vierten Schritt 4 wurde die Produktionsmasse in der Silikonform für 30 Minuten bei einer Temperatur von 98 °C erhitzt.

### Beispiel 2

In Beispiel 2 wurde das erfindungsgemäße Verfahren dazu verwendet, um ein Fleischersatz- oder Fleischimitatprodukt als Imitat von Rostbratwürstchen herzustellen. Die Zerkleinerung von Kräuterseitlingen erfolgte getrennt, einerseits mit einer Wolfscheibe mit Rundlöchern von 8 mm Durchmesser und andererseits durch eine Lochscheibe mit quadratischen Öffnungen von 6 mm mal 6 mm, wobei das Mengenverhältnis der beiden unterschiedlich zerkleinerten Teilmengen 1:1 beträgt. Die Produktionsmasse wurde im zweiten Schritt 2 durch Mischen der unterschiedlich zerkleinerten Kräuterseitlinge (72 Gew%) mit getrocknetem Hühnereieiweiß, Rapsöl, gekochtem Reis, Salz, Zucker, getrocknetem Hühnereieigelb, Gewürze und Wasser erhalten. Die Produktionsmasse wurde im dritten Schritt 3 in einen Wurstschlauch abgefüllt, wobei dieser zur Ausformung der einzelnen Rostbratwürstchen in bestimmten Abständen durch Drehen abgeschnürt wurde. Der Wurstschlauch besteht bevorzugt aus Kunststoff, welche vor dem Verzehr oder vor dem Braten der Rostbratwürstchen zu entfernen ist. Im vierten Schritt 4 wurde der gefüllte Wurstschlauch 30 Minuten bei einer Temperatur von 98 °C erhitzt

### Beispiel 3:

In Beispiel 3 wurde das erfindungsgemäße Verfahren dazu verwendet, um ein Fleischersatz- oder Fleischimitatprodukt als Imitat von Bratstreifen herzustellen. Die Produktionsmasse wurde im zweiten Schritt 2 durch Mischen von zerkleinerten Kräuterseitlingen (76 Gew%) mit gekochtem Reis, getrocknetem Hühnereieiweiß, Rapsöl, Salz und Gewürzen erhalten. Die Produktionsmasse wurde im dritten Schritt 3 in eine Silikonform abgefüllt. Im vierten Schritt 4 wurde die Produktionsmasse in der Silikonform 30 Minuten bei einer Temperatur von 98 °C erhitzt

### Beispiel 4

In Beispiel 4 wurde das erfindungsgemäße Verfahren dazu verwendet, um ein Fleischersatz- oder Fleischimitatprodukt als Imitat von Fischstäbchen herzustellen. Die Produktionsmasse wurde im zweiten Schritt 2 durch Mischung von zerkleinerten Kräuterseitlingen (62 Gew%), gekochtem Reis, Rapsöl, getrocknetem Hühnereieiweiß, Hühnervollei, Salz und Gewürzen erhalten. Die Produktionsmasse wurde im dritten Schritt 3 in eine Silikonform abgefüllt. Im vierten Schritt 4 wurde die Produktionsmasse in der Silikonform 30 Minuten bei einer Temperatur von 98 °C erhitzt.

Die Produktionsmasse kann vor dem Erhitzen in Schritt 4 mit einer Panier versehen werden. Zudem ist es möglich eine Panier erst nach dem Erhitzen der Produktionsmasse aufzutragen. Beispielsweise kann das in Beispiel 1 hergestellte Produkt, wie bei Schnitzelfleisch bekannt, unmittelbar vor dem Braten in der Pfanne paniert werden.

### Texturanalyse

Für zwei unterschiedliche Produktausführungen wurden Textureigenschaften messtechnisch durch das Fraunhofer-Institut für Verfahrenstechnik und Verpackung durch instrumentelle Texturanalyse quantifiziert. Dafür wurden Texturuntersuchungen hinsichtlich Härte, Klebrigkeit, Elastizität, Kohäsion, Gummiartigkeit und Kaufähigkeit vorgenommen. Als Methode wurde dazu die "Textur Profil Analyse" nach Lin et al. ["Texture and Chemical Characteristics of Soy Protein Meat Analog Extruded at High Moisture", Journal of Food Science, Vol. 65, No. 2, 2000] angewandt. Um die Scherkraft der Produktausführungen zu bestimmen, wurde eine Warner Bratzler Klinge herangezogen. Die Produktausführung 1 ist gemäß Beispiel 1 hergestellt. Die Produktausführung 2 ist gemäß Beispiel 2 hergestellt.

Die Zusammensetzung der beiden analysierten Produktausführungen gestaltet sich wie folgt:

| | Produktausführung 1 [g/100g] | Produktausführung 2 [g/100g] |
|---|---|---|
| Kräuterseitling | 77 | 73 |
| Gekochter Reis | 14 | 3 |
| Hühnereieiweiß | 4 | 12 |
| Pflanzenöl | 2 | 5 |
| Salz | 1 | 3 |

Für die "Textur Profil Analyse" wurden alle Proben bis zur Verwendung auf 1 °C gekühlt und vor der Probenvorbereitung auf Raumtemperatur angeglichen. Die rohen Proben zur Produktausführung 1 wurden in zylindrische Kreise mit einem Durchmesser von 25 mm ausgestochen. Die rohen Proben zur Produktausführung 2 wurden in 5 mm dicke Scheiben geschnitten. Für die gebratenen Proben wurden beide Produktausführungen unzerkleinert in einer Pfanne mit etwas Öl auf Stufe 2 auf jeder Seite eine Minute lang gebraten und anschließend wie oben beschrieben zugeschnitten. Eine zylindrische Probe mit 25 mm Durchmesser (SMS P/75) wurde verwendet und zweimal auf 50 % der Probendicke komprimiert. Durch die "Textur Profil Analyse" konnten Härte, Klebrigkeit, Elastizität, Kohäsion, Gummiartigkeit und Kaufähigkeit ausgewertet werden. Die Messparameter sind in folgender Tabelle zusammengefasst:

| | | |
|---|---|---|
| Einstellungen | Test mode: | Compression |
| | Pre-test speed: | 3 mm/s |
| | Test speed: | 1,00 mm/s |
| | Post-test speed: | 3 mm/s |
| | Target mode: | Strain |
| | Distance: | 50 % |
| | Trigger type: | Auto (force) |
| | Trigger force: | 4,1 g |
| | Stop plot at: | Start position |
| Messzelle | 50 kg | |
| Messstempel | SMS P/75 | |

Die Bestimmung der Scherkraft wurde durch eine Warner Bratzler Klinge durchgeführt, alle Proben bis zur Verwendung auf 1 °C gekühlt und vor der Probenvorbereitung auf Raumtemperatur angeglichen wurden.

Die Produktausführung 1 wurde dafür in Quadrate (Abmessungen 19 mm x 19 mm) geschnitten. Die Proben zur Produktausführung 2 wurden in 2 cm lange Stücke geschnitten und anschließend Proben mit einem Durchmesser von 1,27 cm ausgestanzt. Es wurde eine rechteckige Klinge mit Kerbung und eine rechteckige Klinge ohne Kerbung, sowie eine metallische Plattform (HDP/90, Winopal Forschungsbedarf, Ahnbeck) verwendet.

Die Wertebereiche der Scherkraft, Härte, Klebrigkeit, Elastizität, Kohäsion, Gummiartigkeit und Kaufähigkeit wurden in den zwei folgenden Tabellen zusammengefasst.

| | Wertebereiche | |
|---|---|---|
| | Produktausführung 1 (roh) | Produktausführung 1 (gebraten) |
| Scherkraft | 0,674 bis 4,655 | 0,653 bis 1,871 |
| Bewertung: Kraft | | |
| Einheit: [kg] | | |
| Härte | 2814 bis 4471 | 3223 bis 6277 |
| Bewertung: Kraft | | |
| Einheit: [g] | | |
| Klebrigkeit | -44 bis -13 | -53 bis -3 |
| Bewertung: Fläche | | |
| Einheit: [g*sec] | | |
| Elastizität | 75 bis 84 | 51 bis 85 |
| Bewertung: Verhältnis der Distanz | | |
| Einheit: [%] | | |
| Kohäsion | 0,477 bis 0,608 | 0,466 bis 0,751 |
| Bewertung: Verhältnis der Fläche | | |
| Einheit: - | | |
| Gummiartigkeit | 1587 bis 2285 | 1706 bis 3512 |
| Bewertung: Kraft * (Verhältnis der Fläche) | | |
| Einheit: [g] | | |
| Kaufähigkeit | 1216 bis 1903 | 1268 bis 2679 |
| Bewertung: Kraft * (Verhältnis der Fläche) * (Verhältnis der Distanz) | | |
| Einheit: [g] | | |

| | Wertebereiche | |
|---|---|---|
| | Produktausführung 2 (roh) | Produktausführung 2 (gebraten) |
| Scherkraft | 0,495 bis 2,001 | 0,263 bis 1,220 |
| Bewertung: Kraft | | |
| Einheit: [kg] | | |
| Härte | 2215 bis 6362 | 3038 bis 6623 |
| Bewertung: Kraft | | |
| Einheit: [g] | | |
| Klebrigkeit | -7 bis -1 | -6 bis -1 |
| Bewertung: Fläche | | |
| Einheit: [g*sec] | | |
| Elastizität | 70 bis 94 | 81 bis 91 |
| Bewertung: Verhältnis der Distanz | | |
| Einheit: [%] | | |
| Kohäsion | 0,023 bis 0,814 | 0,742 bis 0,802 |
| Bewertung: Verhältnis der Fläche | | |
| Einheit: - | | |
| Gummiartigkeit | 1680 bis 4941 | 2437 bis 5148 |
| Bewertung: Kraft * (Verhältnis der Fläche) | | |
| Einheit: [g] | | |
| Kaufähigkeit | 1357 bis 4603 | 1749 bis 4649 |
| Bewertung: Kraft * (Verhältnis der Fläche) * (Verhältnis der Distanz) | | |
| Einheit: [g] | | |

## Patentansprüche

1. Verfahren zur Herstellung eines fleischlosen Fleischersatz- oder Fleischimitatprodukts, **dadurch gekennzeichnet, dass** im ersten Schritt (1) Fruchtkörper von Speisepilzen zu Stücken zerkleinert werden, wobei zumindest eine Teilmenge der Fruchtkörper auf Stücke mit einer minimalen Erstreckung von 3 mm zerkleinert wird, im zweiten Schritt (2) Zutaten umfassend die zerkleinerten Fruchtkörper von Speisepilzen im Bereich von 40 bis 90 Gew%, Pflanzenöl im Bereich von 2 bis 20 Gew%, eine Stärkequelle, insbesondere gekochter Reis, im Bereich von 2 bis 15 Gew%, tierische und/oder pflanzliche Proteine im Bereich von 4 bis 15 Gew% und Salz im Bereich von 1 bis 3 Gew% zu einer homogenen Produktionsmasse (5) vermischt werden, im dritten Schritt (3) die Produktionsmasse (5) in zumindest eine Form (7) abgefüllt wird, im vierten Schritt (4) die Produktionsmasse (5) in der zumindest einen Form (7) erhitzt wird, sodass sich das pflanzliche oder tierische Protein in der wahlweise geöffneten oder geschlossenen Form verfestigt.

2. Verfahren nach Anspruch 1, **dadurch gekennzeichnet, dass** im ersten Schritt (1) zumindest eine Teilmenge der Fruchtkörper auf Stücke mit einer maximalen Erstreckung von 25 mm zerkleinert wird.

3. Verfahren nach einem der Ansprüche 1 bis 2, **dadurch gekennzeichnet, dass** das Zerkleinern durch Pressen der Fruchtkörper durch zumindest zwei unterschiedliche Lochscheiben erfolgt, wobei jeweils eine Teilmenge der Fruchtkörper durch je eine der Lochscheiben zerkleinert wird.

4. Verfahren nach einem der Ansprüche 1 bis 3, **dadurch gekennzeichnet, dass** eine der Lochscheiben runde Öffnungen mit einem Durchmesser von 5 bis 10 mm aufweist und eine andere der Lochscheiben rechteckige Öffnungen mit Kantenlängen von 3 bis 15 mm aufweist.

5. Verfahren nach einem der Ansprüche 1 bis 4, **dadurch gekennzeichnet, dass** die Form (7) ausgewählt ist aus einem Kunststoffschlauch, einem Kunststoffbeutel oder einer Gießform aus Silikon.

6. Verfahren nach einem der Ansprüche 1 bis 5, **dadurch gekennzeichnet, dass** das Mischen in einem Mischer (6) in einem Temperaturbereich von 10 bis 20 °C und einer Zeit von 1 bis 10 Minuten erfolgt und das Erhitzen in der Form (7) in einem Temperaturbereich von 80 bis 100 °C und einer Zeit von 10 bis 45 Minuten erfolgte.

7. Fleischloses Fleischersatz- oder Fleischimitatprodukt hergestellt nach einem der Ansprüche 1 bis 6.

8. Fleischersatz- oder Fleischimitatprodukt nach Anspruch 7, **dadurch gekennzeichnet, dass** zumindest eine Teilmenge der zerkleinerten Fruchtkörper als Stücke mit einer minimalen Erstreckung von zumindest 3 mm und einer maximalen Erstreckung von maximal 25 mm vorliegt.

9. Fleischersatz- oder Fleischimitatprodukt nach einem der Ansprüche 7 oder 8, **dadurch gekennzeichnet, dass** die zerkleinerten Fruchtkörper als zumindest zwei unterschiedlich stark zerkleinerte Teilmengen vorliegen.

10. Fleischersatz- oder Fleischimitatprodukt nach einem der Ansprüche 7 bis 9, **dadurch gekennzeichnet, dass** zumindest eine Teilmenge der zerkleinerten Fruchtkörper aus zylindrischen Fruchtkörperstücken gebildet ist, die einen Durchmesser von 5 bis 10 mm aufweisen und eine maximale Erstreckung von 3 bis 15 mm.

11. Fleischersatz- oder Fleischimitatprodukt nach einem der Ansprüche 7 bis 10, **dadurch gekennzeichnet, dass** zumindest eine Teilmenge der zerkleinerten Fruchtkörper aus würfeligen oder quaderförmigen Fruchtkörperstücken gebildet ist, die eine quadratische Grundfläche von 3 x 3 mm bis 9 x 9 mm aufweisen und eine Länge von 5 bis 25 mm.

12. Fleischersatz- oder Fleischimitatprodukt nach einem der Ansprüche 7 bis 11, **dadurch gekennzeichnet, dass** der Speisepilz ein Pilz der Gattung Pleurotus, insbesondere der Art Pleurotus eryngii ist.

13. Fleischersatz- oder Fleischimitatprodukt nach einem der Ansprüche 7 bis 12, **dadurch gekennzeichnet, dass** das Produkt bei der Texturprofilanalyse folgende Werte aufweist:
- Scherkraft im Bereich von 0,2 bis 2 kg,
- Härte im Bereich von 2 bis 7 kg,
- Elastizität im Bereich von 50 bis 95 %,
- Kaufähigkeit im Bereich von 1 bis 5 kg.

14. Fleischersatz- oder Fleischimitatprodukte nach einem der Ansprüche 7 bis 13, **dadurch gekennzeichnet, dass** der Speisepilz Kräuterseitling (Pleurotus eryngii) ist.

## Claims

1. Method for producing a meat-free meat-substitute or imitation-meat product, **characterized in that** fruit bodies of edible mushrooms are broken up into pieces in the first step (1), at least one portion of the fruit bodies being broken up into pieces that have a minimum extension of 3 mm, ingredients comprising the broken-up fruit bodies of edible mushrooms in the range of from 40 to 90 wt.%, plant oil in the range of from 2 to 20 wt.%, a starch source, in particular cooked rice, in the range of from 2 to 15 wt.%, animal and/or plant proteins in the range of from 4 to 15 wt.% and salt in the range of from 1 to 3 wt.% are mixed to form a homogenous production compound (5) in the second step (2), the production compound (5) is filled into at least one mold (7) in the third step (3), the production compound (5) is heated in the at least one mold (7) in the fourth step (4) such that the plant or animal protein solidifies in the optionally open or closed mold.

2. Method according to claim 1, **characterized in that**, in the first step (1), at least one portion of the fruit bodies are broken up into pieces that have a maximum extension of 25 mm.

3. Method according to either claim 1 or claim 2, **characterized in that** the fruit bodies are broken up by being pressed through at least two different perforated disks, each perforated disk breaking up one portion of the fruit bodies.

4. Method according to any of claims 1 to 3, **characterized in that** one of the perforated disks comprises round openings that have a diameter of from 5 to 10 mm and another of the perforated disks comprises rectangular openings that have edge lengths of from 3 to 15 mm.

5. Method according to any of claims 1 to 4, **characterized in that** the mold (7) is selected from a plastics tube, a plastics bag or a silicone casting mold.

6. Method according to any of claims 1 to 5, **characterized in that** the mixing takes place in a mixer (6) within a temperature range of from 10 to 20°C and in a time of from 1 to 10 minutes and the heating in the mold (7) takes place within a temperature range of from 80 to 100°C and in a time of from 10 to 45 minutes.

7. Meat-free meat-substitute or imitation-meat product produced according to any of claims 1 to 6.

8. Meat-substitute or imitation-meat product according to claim 7, **characterized in that** at least one portion of the broken-up fruit bodies are in the form of pieces that have a minimum extension of at least 3 mm and a maximum extension of at most 25 mm.

9. Meat-substitute or imitation-meat product according to either claim 7 or claim 8, **characterized in that** the broken-up fruit bodies are in the form of at least two portions that are broken up to different degrees.

10. Meat-substitute or imitation-meat product according to any of claims 7 to 9, **characterized in that** at least one portion of the broken-up fruit bodies is formed from cylindrical fruit body pieces that have a diameter of from 5 to 10 mm and a maximum extension of from 3 to 15 mm.

11. Meat-substitute or imitation-meat product according to any of claims 7 to 10, **characterized in that** at least one portion of the broken-up fruit bodies is formed from cubic or cuboid fruit body pieces that have a square base area of from 3 x 3 mm to 9 x 9 mm and a length of from 5 to 25 mm.

12. Meat-substitute or imitation-meat product according to any of claims 7 to 11, **characterized in that** the edible mushroom is a mushroom of the Pleurotus genus, in particular of the Pleurotus eryngii species.

13. Meat-substitute or imitation-meat product according to any of claims 7 to 12, **characterized in that** the product has the following values in the texture profile analysis:
- shear force in the range of from 0.2 to 2 kg,
- hardness in the range of from 2 to 7 kg,
- elasticity in the range of from 50 to 95%,
- absorbency in the range of from 1 to 5 kg.

14. Meat-substitute or imitation-meat products according to any of claims 7 to 13, **characterized in that** the edible mushroom is king oyster mushroom (Pleurotus eryngii).

## Revendications

1. Procédé de production d'un produit sans viande de substitution à la viande ou d'imitation de viande, **caractérisé en ce que**, dans une première étape (1), des sporophores de champignons comestibles sont broyés en morceaux, au moins une quantité partielle des sporophores étant broyée en morceaux avec une étendue minimale de 3 mm ; dans une deuxième étape (2), des ingrédients comprenant les sporophores broyés de champignons comestibles dans une plage de 40 à 90 % en poids, de l'huile végétale dans la plage de 2 à 20 % en poids, une source d'amidon, en particulier du riz cuit, dans la plage de 2 à 15 % en poids, des protéines animales et/ou végétales dans la plage de 4 à 15 % en poids, et du sel dans la plage de 1 à 3 % en poids sont mélangés à une masse de production homogène (5) ; dans une troisième étape (3), la masse de production (5) est mise en emballage au moins sous une forme (7); dans une quatrième étape (4), la masse de production (5) est chauffée dans l'au moins une forme (7), de sorte que la protéine végétale ou animale se solidifie dans la forme sélectivement ouverte ou fermée.

2. Procédé selon la revendication 1, **caractérisé en ce que**, dans la première étape (1), au moins une quantité partielle des sporophores est broyée en morceaux avec une étendue maximale de 25 mm.

3. Procédé selon l'une des revendications 1 à 2, **caractérisé en ce que** le broyage par pressage des sporophores a lieu au moyen d'au moins deux disques perforés différents, une quantité partielle respective des sporophores étant broyée par un disque perforé correspondant.

4. Procédé selon l'une des revendications 1 à 3, **caractérisé en ce que** l'un des disques perforés présente des ouvertures rondes d'un diamètre de 5 à 10 mm, et l'autre des disques perforés présente des ouvertures rectangulaires avec des côtés d'une longueur 3 à 15 mm.

5. Procédé selon l'une des revendications 1 à 4, **caractérisé en ce que** la forme (7) est choisie parmi un conduit en plastique, un sac plastique ou un moule en silicone.

6. Procédé selon l'une des revendications 1 à 5, **caractérisé en ce que** le mélange a lieu dans un mélangeur (6) à une température dans la plage de 10 à 20 °C et sur une durée de 1 à 10 minutes, et le réchauffement dans la forme (7) a lieu à une température dans la plage de 80 à 100 °C et sur une durée de 10 à 45 minutes.

7. Produit sans viande de substitution à la viande ou d'imitation de viande fabriqué selon l'une des revendications 1 à 6.

8. Produit de substitution à la viande ou d'imitation de viande selon la revendication 7, **caractérisé en ce qu'**au moins une quantité partielle des sporophores broyés en morceaux d'une étendue minimale d'au moins 3 mm et d'une étendue maximale d'au plus 25 mm est présente.

9. Produit de substitution à la viande ou d'imitation de viande selon l'une des revendications 7 ou 8, **caractérisé en ce que** les sporophores broyés sont présents sous la forme d'au moins deux quantités partielles différentes fortement broyées.

10. Produit de substitution à la viande ou d'imitation de viande selon l'une des revendications 7 à 9, **caractérisé en ce qu'**au moins une quantité partielle des sporophores broyés est constituée de morceaux de sporophores cylindriques qui présentent un diamètre de 5 à 10 mm et une étendue maximale de 3 à 15 mm.

11. Produit de substitution à la viande ou d'imitation de viande selon l'une des revendications 7 à 10, **caractérisé en ce qu'**au moins une quantité partielle des sporophores broyés est constituée de morceaux de sporophores cubiques ou parallélépipédiques qui présentent une surface quadratique de 3 x 3 mm à 9 x 9 mm et une longueur de 5 à 25 mm.

12. Produit de substitution à la viande ou d'imitation de viande selon l'une des revendications 7 à 11, **caractérisé en ce que** le champignon comestible est un champignon du genre Pleurotus, en particulier de la variété Pleurotus eryngii.

13. Produit de substitution à la viande ou d'imitation de viande selon l'une des revendications 7 à 12, **caractérisé en ce que** le produit, lors de l'analyse de profil de texture, présente les valeurs suivantes :
- une force de cisaillement dans la plage de 0,2 à 2 kg,
- une dureté dans la plage de 2 à 7 kg,
- une élasticité dans la plage de 50 à 95 %,
- une capacité de mastication dans la plage de 1 à 5 kg.

14. Produit de substitution à la viande ou d'imitation de viande selon l'une des revendications 7 à 13, **caractérisé en ce que** le champignon comestible est un pleurote du panicaut (Pleurotus eryngii).
